# EUROPEAN PATENT SPECIFICATION

(11) **EP 1 487 261 B1**
(45) Date of publication and mention of the grant of the patent: **27.08.2008**
(21) Application number: 03744148.2
(22) Date of filing: 04.03.2003
(51) Int. Cl.: A01K 67/027

(54) **METHODS AND COMPOSITIONS FOR PHARMACOLOGICAL AND TOXICOLOGICAL EVALUATION OF TEST AGENTS**
VERFAHREN UND ZUSAMMENSETZUNGEN FÜR DIE PHARMAKOLOGISCHE UND TOXIKOLOGISCHE AUSWERTUNG VON TESTSUBSTANZEN
PROCEDES ET COMPOSITIONS D'EVALUATION PHARMACOLOGIQUE ET TOXICOLOGIQUE D'AGENTS D'ESSAI

(30) Priority: 05.03.2002 US 361890 P
(43) Date of publication of application: 22.12.2004
(73) Proprietor: MCW Research Foundation, Incorporated, Milwaukee Wisconsin 53226 (US); PHYSIOGENIX, INC., Milwaukee, Wisconsin 53226 (US)
(72) Inventor: JACOBS, Howard, J., Brookfield, WI 53005 (US); ROMAN, Richard, J., Brookfield, WI 53005 (US); NYE, Steven, H., Mequon, WI 53092 (US)
(74) Representative: Duckworth, Timothy John
(86) International application number: PCT/US2003/006421
(87) International publication number: WO 2003/075838

(56) References cited:
- WOLFF G L: "SOME GENETIC CONSIDERATIONS FOR THE DESIGN OF BETTER MAMMALIAN ASSAY SYSTEMS FOR THE DETECTION OF CHEMICAL MUTAGENS AND CARCINOGENS" JOURNAL OF ENVIRONMENTAL PATHOLOGY AND TOXICOLOGY, vol. 1, no. 2, 1997, page RECD, XP009043925 ISSN: 0146-4779
- VAN ZUTPHEN L F M: "Toxicity testing and genetic quality control" JOURNAL OF EXPERIMENTAL ANIMAL SCIENCE, vol. 35, no. 5-6, 1993, pages 202-209, XP009043920 ISSN: 0939-8600
- FESTING MICHAEL F W: "Genetic variation in outbred rats and mice and its implications for toxicological screening" JOURNAL OF EXPERIMENTAL ANIMAL SCIENCE, vol. 35, no. 5-6, 1993, pages 210-220, XP009043919 ISSN: 0939-8600
- MARCOTTE E R ET AL: "DNA microarrays in neuropsychopharmacology" TRENDS IN PHARMACOLOGICAL SCIENCES, ELSEVIER TRENDS JOURNAL, CAMBRIDGE, GB, vol. 22, no. 8, 1 August 2001 (2001-08-01), pages 426-436, XP004296254 ISSN: 0165-6147
- PHELAN J.P.: 'Genetic variability and rodent models of human aging' EXPERIMENTAL GERONTOLOGY vol. 27, 1992, pages 147 - 159, XP002953250

## Description

### BACKGROUND OF THE INVENTION

Pharmaceuticals often exhibit wide differences in potency and efficacy among individuals, and drugs, chemicals and food products often can have unexpected toxic effects on humans. Reliable methods for predicting such effects have eluded the pharmaceutical and drug discovery industry as well as those charged with evaluating food and product safety.

For example, early-stage evaluation of candidate therapeutic agents typically involves testing non-human subjects without considering whether a subject's genotype or the genotype of the model system might have an effect on therapeutic efficacy or adverse side effects. Late-stage clinical studies typically involve testing ten to twenty-fold more subjects than early-stage trials. Once-promising candidate therapeutic agents can fail in late-stage clinical studies due to the increased likelihood that one of the subjects has a genotype that responds poorly or exhibits adverse side effects. Genetic heterogeneity is highly likely among the numerous patients that might receive a therapeutic agent, and an agent that adversely affects even a rare genotype can represent an unacceptable risk of harm, perhaps resulting in discontinued development or withdrawal from the market of an already approved drug.

### BRIEF SUMMARY OF THE INVENTION

The invention relates to methods and materials useful for evaluating test agents. In particular, the invention pertains to collections of rats and their use to evaluate effects of test agents in different genotypes.

The invention is based, in part, on the combinatorial mating of inbred rats to yield collections of offspring that exhibit genetic diversity (e.g., in particular metabolic enzymes and pathways). The invention features collections of combinatorially bred animals useful for evaluating effects of test agents, including candidate therapeutic molecules and putative allergens, toxins, chemicals or food products in different genotypes.

Thus, the present invention provides a collection of rats comprising combinatorially bred offspring of at least four genetically different inbred rat strains, the inbred strains having been mated in each different pair-wise combination to produce the collection, where the inbred strains comprise the Fischer 344, Brown Norway, Lewis, and Wistar Kyoto strains and said offspring comprising six or more F₁ individuals from each different mating of said inbred strains.

The disclosed methods for evaluating effects of test agents in different genotypes can save considerable time and money in the development of new therapeutic molecules, and can improve the accuracy of product, food and environmental safety determinations.

The present invention also provides a collection of cDNA molecules made by reverse transcription of RNA isolated from each member of a collection of rats of the invention.

The invention also features the use of microarrays that have nucleic acids, preferably cDNAs, derived from all the genes expressed in each rat of a collection of rats of the invention attached to a solid support. The invention further provides therefore a cDNA microarray comprising a collection of labeled cDNA molecules made by reverse transcription of RNA isolated from each member of a collection of rats of the invention, wherein said cDNA molecules are hybridized to gene specific DNA, cDNA, or oligonucleotide targets attached to a single solid substrate in a non-random manner. RNAs are isolated from two individuals from the collection or pooled from two different strains of the combinatorial mating labeled with two different color fluorescent dyes and hybridized together with the nucleic acid on the microarray to find those genes that are differentially expressed between the strains. This approach allows one to use the combinatorially bred offspring to identify individual genes contributing to the differential response to a toxin or compound.

In one aspect, the invention features collections of rats including combinatorially bred offspring of at least 4 genetically different inbred rat strains. In another embodiment, the collection includes combinatorially offspring of at least six or at least eight or at least 10 genetically different inbred rat strains. The offspring include 6 or more F₁ individuals, or 8 or more F₁, individuals from each different mating of the inbred strains. In some embodiments, the offspring include 12 or more F₁ individuals from each different mating of the inbred strains. In some embodiments the inbred rodent strains are inbred strain F344. In some embodiments, the inbred strains collectively include at least 2 alleles for a preselected genetic locus (e.g., a locus that encodes a cytochrome P-450 enzyme). In some embodiments, the inbred strains are selected to maximize the number of alleles at one or more preselected genetic loci (e.g., genetic loci that encode members of a class of toxicologically relevant enzymes, such as cytochrome P-450 enzymes).

In another aspect, the invention features nucleic acid, preferably RNA isolated from each member of the above-described collection of rats. In a preferred embodiment, the RNA from pairs of rats from the above described collection or pooled samples representative of the strains from each combination is hybridized to cDNA microarrays. The collection of rats includes combinatorially bred offspring of at least 4 genetically different inbred rats strains, and the offspring include 6 or more F₁ individuals from each different mating of the inbred strains.

Also referred to is a method for making a collection of rats.

The method involves combinatorially mating at least 4 genetically different inbred rat strains, and pooling 6 or more F₁, individuals from each different mating of the inbred strains. In some embodiments, the inbred rat strains collectively exhibit maximal genetic heterogeneity across the genome. In some embodiments, the inbred rat strains collectively exhibit maximal genetic heterogeneity across one or more chromosomes. In some embodiments, the inbred rat strains collectively exhibit maximal genetic heterogeneity at one or more genetic loci (e.g., genetic loci that encode a class of toxicologically relevant enzymes, such as the cytochrome P-450 class). In another embodiment, the inbred rat model is selected to exhibit maximal diversity in response to a drug or toxic agent or in susceptibility and resistance to develop particularly symptoms of disease, cancer, heart disease, diabetes or stroke.

In another embodiment, the model is selected to minimize diversity at a selected set of genes and maximize remaining overall background genomic diversity.

In another aspect, the invention features a method for evaluating a test agent, said method comprising administering said test agent to each member of a collection of rats of the invention and measuring an effect of said test agent on said rats. In some embodiments, measuring the effect of a test agent involves preparing cDNA from RNA isolated from the rats and hybridizing the cDNA from RNA isolated from the rats and hybridizing the cDNA to a nucleic acid microarray.

Other features and advantages of the invention will be apparent from the following detailed description and from the claims.

Unless otherwise defined, all technical and scientific terms used herein have the meaning commonly understood by one of ordinary skill in the art to which this invention belongs. In case of conflict, the present specification, including definitions, will control. The disclosed materials, methods, and examples are illustrative only and not intended to be limiting. Skilled artisans will appreciate that methods and materials similar or equivalent to those described herein can be used to practice the invention.

### BRIEF DESCRIPTION OF THE SEVERAL VIEWS OF THE DRAWINGS

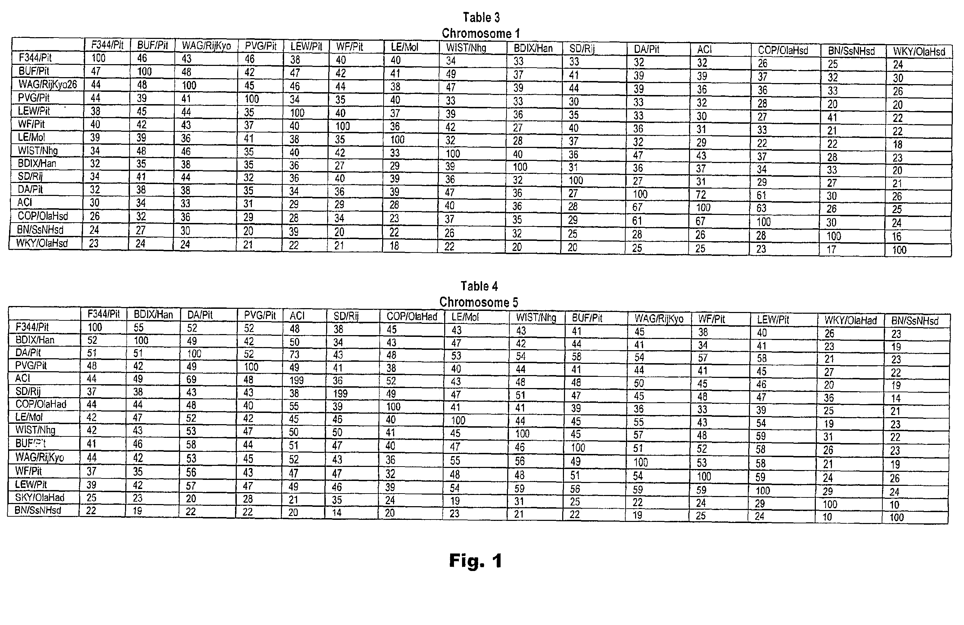

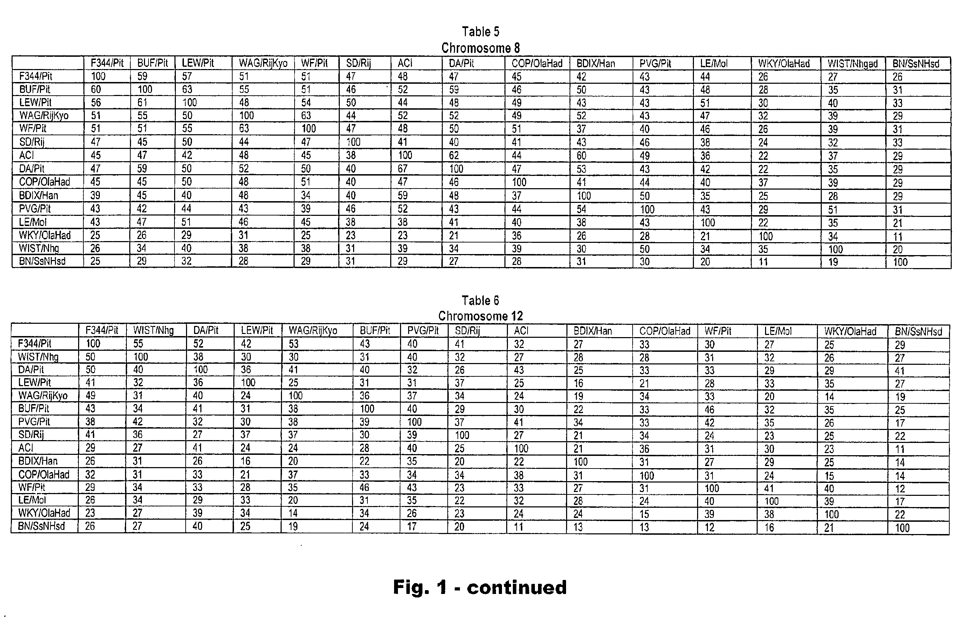

Fig. 1 is a set of tables showing the percentage of genetic similarity on a particular chromosomes between pairs of rodent strains.

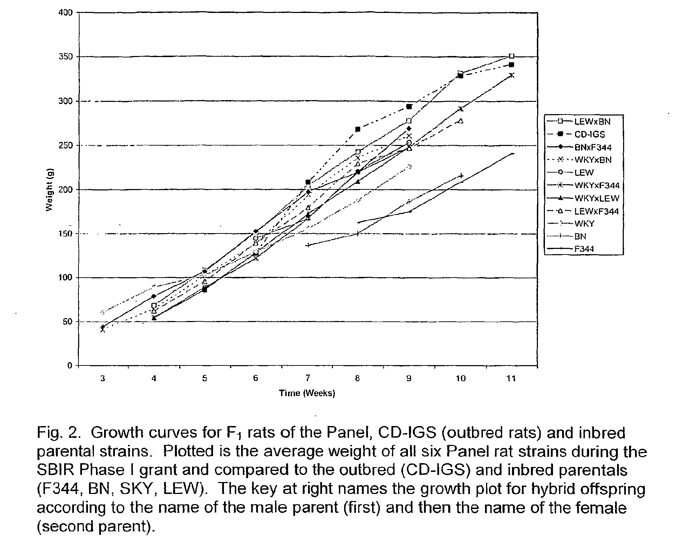

Fig 2 is a set of growth curves for all rats in the Phase I studies. Plotted is the average weight of all six Panel rat strains compared to the outbred (CD-IGS) and inbred parentals (F344, BN, SKY, LEW). The key at right names the growth plot for hybrid offspring according to the name of the male parent (first) and then the name of the female (second parent).

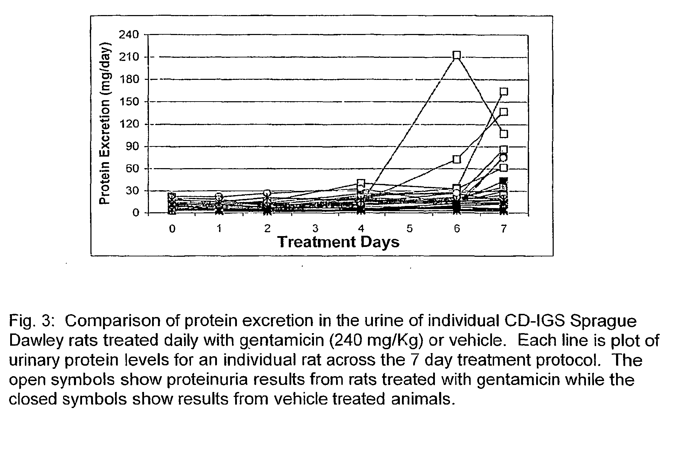

Fig. 3 compares protein excretion in urine of individual Sprague Dawley rats treated daily with gentamicin (240 mg/Kg) or vehicle. Each line is a plot of urinary protein levels for an individual rat across the 7 day treatment protocol. The open symbols show proteinuria results from rats treated with gentamicin while the closed symbols show results from vehicle treated animals.

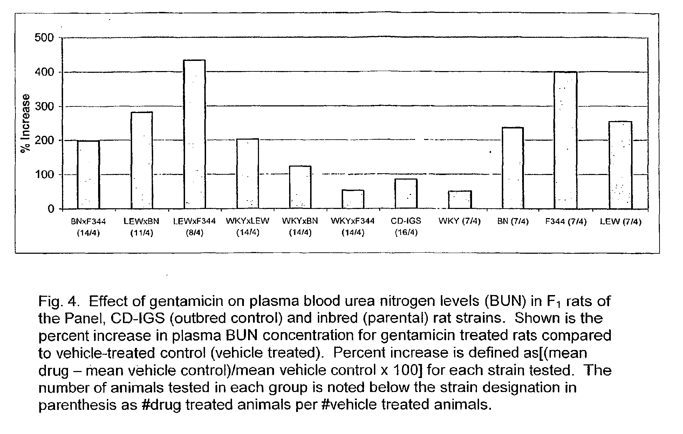

Fig. 4 demonstrates the effect of gentamicin on plasma blood urea nitrogen (BUN) levels in Panel, CD-IGS (outbred control) and inbred (parental) rat strains. Shown is the percent increase in plasma BUN concentration for gentamicin treated rats compared to vehicle-treated control (vehicle treated). Percent increase is defined as[(mean drug- mean vehicle control)/mean vehicle control x 100] for each strain tested. The number of animals tested in each group is noted below the strain designation in parenthesis as #drug treated animals per#vehicle treated animals.

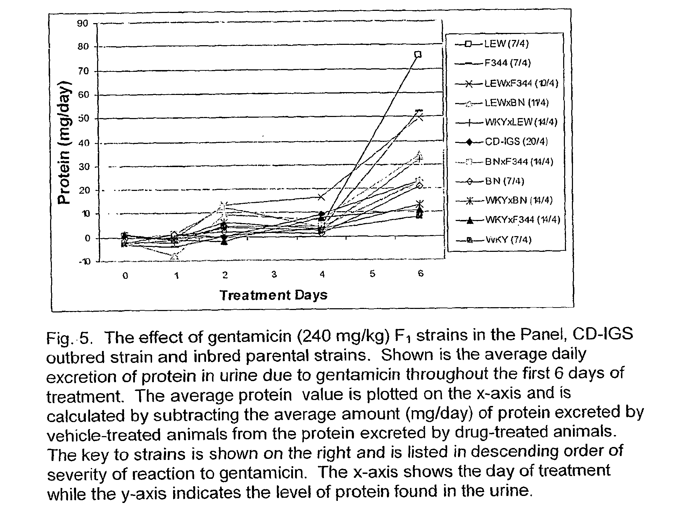

Fig. 5 illustrates the effect of gentamicin (240 mg/kg) for all strains in Phase I studies. Shown is the average daily excretion of protein in urine due to gentamicin throughout the first 6 days of treatment. The average protein value is plotted on the x-axis and is calculated by subtracting the average amount (mg/day) of protein excreted by vehicle-treated animals from the protein excreted by drug-treated animals. The key to strains is shown on the right and is listed in descending order of severity of reaction to gentamicin. The x-axis shows the day of treatment while the y-axis indicates the level of protein found in the urine.

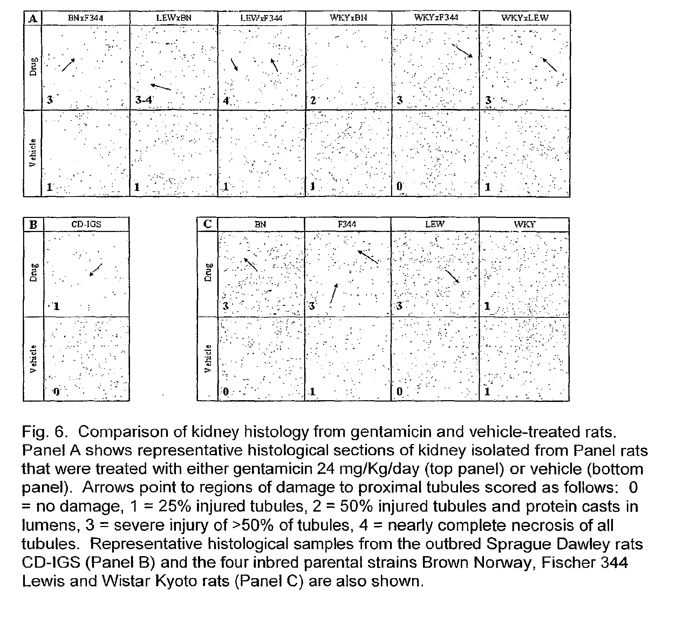

Fig. 6 illustrates comparison of kidney histology from gentamicin and vehicle-treated rats. Panel A shows representative histological sections of kidney isolated from Panel rats that were treated with either gentamicin 240 mg/Kg/day (top panel) or vehicle (bottom panel). Arrows point to regions of the proximal tabules damage scored as follows: 0 = no damage, 1 = 25% injured tubules, 2 = 50% injured tubules and protein casts in lumens, 3 = severe injury of >50% of tubules, 4 = nearly complete necrosis of all tubules. Representative histological samples from the outbred Sprague Dawley rat CD-IGS (Panel B) and the four inbred parental strains Brown Norway, Fischer 344, Lewis and Wistar Kyoto rats (Panel C) are also shown.

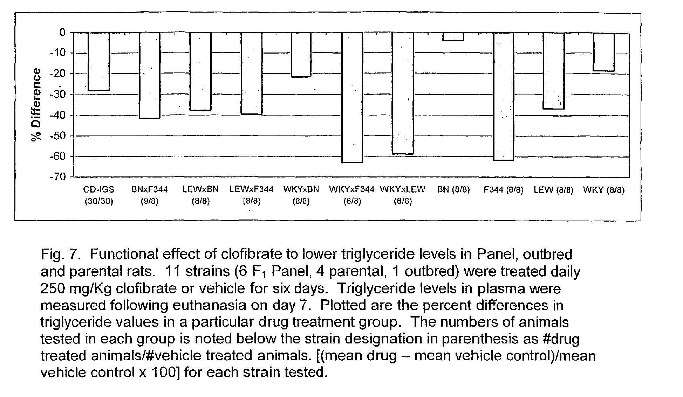

Fig. 7 illustrates functional effect of clofibrate to lower triglyceride levels in the F₁ strains of the Panel, outbred Sprague Dawley (CD-ICS) and parental strains, Brown Norway, Fischer 344, Lewis and Wistar Kyoto rats. 11 strains (6 Panel, 4 parental, 1 outbred) were either treated with six oral injections of 250 mg/Kg clofibrate or HPMC as vehicle. Triglyceride levels in plasma were measured following euthanasia on day 7. Plotted are the percent differences in triglyceride values in a particular drug treatment group. The numbers of animals tested in each group is noted below the strain designation in parenthesis as #drug treated animals/#vehicle treated animals. [(mean drug- mean vehicle control)/mean vehicle control x 100] for each strain tested.

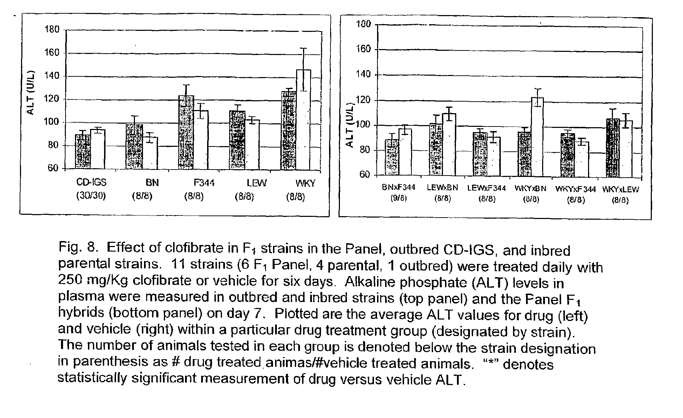

Fig. 8 illustrates the functional effect of clofibrate on the F₁ strains in the Panel, outbred and parental strains, Brown Norway, Fischer 344, Lewis and Wistar Kyoto rats. 11 strains (6 Panel, 4 parental, 1 outbred) were either treated with six oral injections of 250 mg/Kg clofibrate or HPMC has vehicle. Alanine transferase (ALT) levels in plasma an index of liver damage were measured in outbred and inbred strains (top panel) and the Panel F₁ hybrids (bottom panel) following euthanasia on day 7. Plotted are the average ALT values for drug (left) and vehicle (right) within a particular drug treatment group (designated by strain). The number of animals tested in each group is denoted below the strain designation in parenthesis as # drug treated animas/#vehicle treated animals. "*" denotes statistically significant measurement of drug versus vehicle ALT.

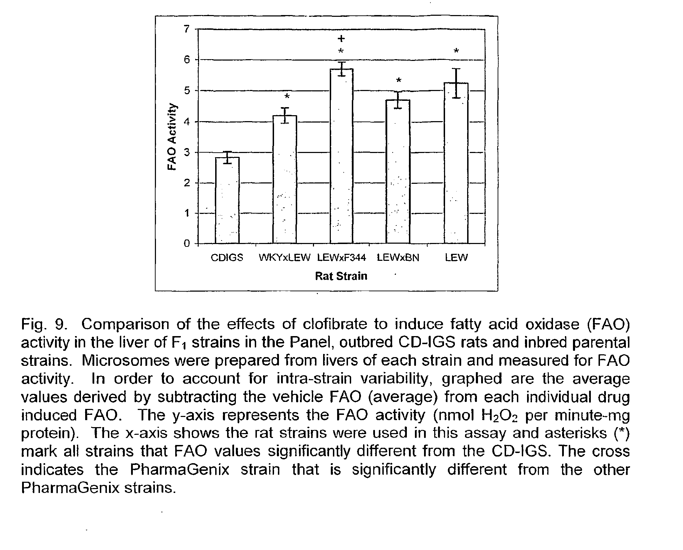

Fig. 9 is a comparison of the effects of clofibrate to induce fatty acid oxidase (FAO) activity in the liver of outbred Sprague Dawley rats, inbred parental strains (Lewis) and F₁ strains of the Panel. Peroxisomal fractions from livers of each strain were measured for FAO activity. In order to account for intra-strain variability, graphed are the average values derived by subtracting the vehicle FAO (average) from each individual drug induced FAO. The y-axis represents the FAO activity (nmol H₂O₂ per minute-mg peroximal protein). The x-axis shows the rat strains were used in this assay and asterisks (*) mark all strains that FAO values significantly different from the CD-IGS. The cross indicates the Panel strain that is significantly different from the other Panel strains.

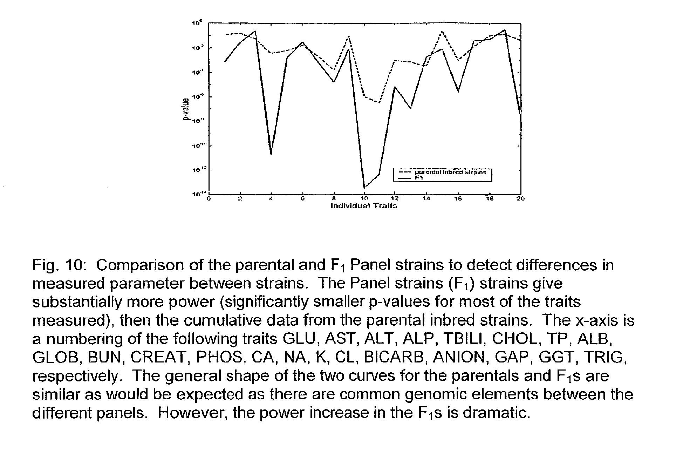

Fig. 10 compared the power of parental strains versus use of the F₁ Panel to detect strain differences in drug responses measured in the present study. The Panel strains (F₁) strains give substantially more power (significantly smaller p-values for most of the traits measured), than the cumulative data from the parental inbred strains. The x-axis is a numbering of the following measured traits GLU, AST, ALT, ALP, TBILI, CHOL, TP, ALB, GLOB, BUN, CREAT, PHOS, CA, NA, K, CL, BICARB, ANION, GAP, GGT, TRIG, respectively. The general shape of the two curves for the parentals and F₁s are similar as would be expected as there are common genomic elements between the different panels. However, the increase in power of the study design using F₁ hybrid rats dramatic.

### DETAILED DESCRIPTION

The invention relates to the combinatorial mating of inbred rats to yield a collection of offspring that exhibits genetic diversity (e.g., in a metabolic enzyme or pathway). Such a collection of combinatorially bred offspring can be used to evaluate differences in biologic responses to test agents based on different genotypes. Test agents can include candidate therapeutic molecules, putative allergens or toxins, foods and other consumer products (e.g., cosmetics, hygiene products, and textiles) and environmental chemicals.

### Combinatorially bred rodents

In one aspect, the invention provides a rat collection (i.e., a number of rats that make up and are considered as a unit). Such a collection includes 6 or more F₁ offspring from each possible combinatorial mating of at least 4 genetically different inbred strains. In another embodiment, the collection includes combinatorially offspring of at least six or at least eight or at least 10 genetically different inbred rat strains.

Such a collection is made by mating the parental strains in each different pair-wise combination. For example, if parent strains A, B, C and D are to be used, the different pair-wise matings are: AxB, AxC, AxD, BxC, BxD and CxD. The F₁ offspring from each mating are pooled to make the collection of combinatorially bred offspring. Offspring from each different pair-wise mating can be obtained from one or more litters, and can be obtained from litters produced by the same or different individual parents. Since F₁ rats from a given mating of two inbred strains are genetically identical, the offspring can be pooled by, for example, placing 6 or more F₁ offspring from each mating of two particular inbred strains in the same rat containment facility.

The genetic diversity of parent strains, and thereby the offspring produced by their combinatorial mating, can be optimized by analyzing genetic markers in candidate parental strains. Using genetic marker analyses, those skilled in the art can identify parent strains that, considered collectively, have: 1) a certain degree of genetic identity or heterogeneity throughout the genome; 2) a certain degree of genetic identity or heterogeneity on one or more particular chromosomes; or 3) a certain number of alleles at one or more preselected genetic loci.

In one embodiment, one can identify and combinatorially mate parental strains that, considered collectively, exhibit the greatest overall genetic diversity to produce a collection of offspring that exhibits maximal diversity across a genome. Such a collection can be particularly useful for evaluating tests agents for which no genetic target is known or suspected.

In another embodiment, one can identify and combinatorially mate parental strains that, considered collectively, exhibit the greatest genetic diversity on one or more particular chromosomes to produce a collection of offspring that exhibits maximal diversity on one or more particular chromosomes (e.g., X or autosomal chromosomes).

In yet another embodiment, one can identify and combinatorially mate parental strains that, considered collectively, exhibit the greatest number of different alleles at one or more particular genetic loci to produce a collection of offspring that exhibits maximal diversity at one or more particular genetic loci (e.g., loci thought to be involved in metabolizing a test agent).

Genetic loci targeted for diversification can include, for example, a genetic locus that encodes a member of a class of enzymes important in the metabolism of drugs and toxins.

Differences in the activity of such enzymes impact on the bioavailability and metabolism of drugs and chemicals, thereby affecting the blood levels, efficacy and toxicity of these agents. Classes of enzymes important in drug metabolism include, for example, the cytochrome P-450 class, the cytochrome P-450 reductase class, the flavin-containing monooxygenase class, the peroxidase class, the epoxide hydrolase class, the arylesterase class, the carboxylesterase class, the acetylesterase class, the cholinesterase class, the amidase class, the alcohol dehydrogenase class, the aldehyde reductase class, the ketone reductase class, the aldehyde dehydrogenase class, the aldehyde oxidase class, the glucuronosyltransferase class, the aryl sulfotransferase class, the hydroxysteroid sulfotransferase class, the estrone sulfotransferase class, the bile salt sulfotransferase class, the methyl transferase class, the N-acetyl transferase class, the ATP-dependent acid:CoA ligase class, the N-acyltransferase class, the glutathione S-alkyltransferase class, the glutathione S-aryltransferase class, the glutathione S-aralkyltransferase class, the glutathione S-alkenetransferase class, the glutathione S-epoxidetransferase class, the glutathione S-aryl epoxidetransferase class, and the rhodanese class and other enzymes related to metabolism of drugs and chemicals that here to date have not yet been identified, but will be known now that the rat genome is being sequenced.

In other embodiments, a genetic locus targeted for diversification encodes a disease-related enzyme, e.g., an enzyme which, when abnormal or abnormally expressed, causes or predisposes an individual to a disease state. Disease-related enzymes can represent potential targets for therapeutic agents. Exemplary disease-related enzymes include angiotensin converting enzyme (associated with hypertension), human leukocyte antigens (associated with e.g., autoimmune diseases such as insulin dependent (type I) or independent (type II) diabetes and multiple sclerosis; cancers such as Hodgkin disease; and infectious diseases, such as tuberculosis and AIDS), P53 (associated with various carcinomas), BRCA1 and BRCA2 (associated with breast cancers), and APP (associated with Alzheimer's disease).

Parental strains can be selected, for example, by using nuclear magnetic resonance, mass spectroscopic analysis and other proteomic approaches to identify small molecule or protein biomarkers in urine and plasma samples that predict strain differences in the toxicity or efficacy of various test agents, or by analyzing polymorphic genetic markers ("markers"). Useful genetic markers include RFLP (Restriction Fragment Length Polymorphisms), SNP (Single Nucleotide Polymorphisms), RAPD (Random Amplified Polymorphic DNA), AFLP (Amplified Fragment Length Polymorphisms), and microsatellite repeats or any other means to determine a sequence variant-which is the basis of all genetic markers. SSLP (Simple Sequence Length Polymorphism) markers are particularly useful genetic markers for analyzing candidate parent strains. For optimizing diversity at a particular genetic locus, markers that are more closely linked (physically or genetically) to the locus of interest are preferable to markers that are less closely linked to the locus.

SSLP markers reportedly are highly mutable, while the flanking DNA is much less mutable. Thus, SSLP allele sizes can be useful to infer whether particular regions of different genomes are the same or different. Individual SSLP markers provide a limited amount of information, but a series of SSLP markers exhibiting identity between different genomes is suggestive that the region of the genome inclusive of the conserved markers is also identical in sequence.

The allele sizes of more than 4,300 of the 5,200 known SSLP markers in the rat genome have been analyzed in 48 commonly studied inbred rat strains. SSLP alleles differing in length by at least 2 base pairs' occur at a rate of 46%. SSLP loci exhibit between 2 and 13 alleles, 6 on average. Closely related strains derived from the same progenitor have multiple long haplotypes (i.e., >10 SSLP markers having the same allele size). The greater the number of polymorphisms between two strains, the fewer the number of haplotypes (≥3 SSLP markers having same allele size) in common. Thus, linkage disequilibrium can be used to study inbred strains and the degree of genetic diversity between strains.

Computer software can be used to discern patterns based on SSLP polymorphisms found within specific regions of a genome. For example, the ACP Haplotyper computer program can be used to compare SSLP allele sizes across inbred strains (e.g., the 48 strains characterized in the rat Allele Characterization Project) with each other, and with a hypothetical "ancestor strain" having the most common allele size at each locus. ACP Haplotyper can order alleles according to a variety of genetic and physical maps, allowing an investigator to visualize regions of conservation between strains and enabling inter-strain comparisons, evolutionary inferences and other practical benefits such as map placement evaluation. ACP Haplotyper is web-based and uses a Perl (Practical Extraction and Report Language) CGI (Common Gateway Interface) with an Oracle 8i (Oracle Corp.) database to handle the storage of the allele and marker data. The ACP Haplotyper computer program is available from the Department of Physiology and the Human and Molecular Genetics Center via the Rat Genome Database, Medical College of Wisconsin, Milwaukee, WI, USA.

In some embodiments, one can combinatorially mate parental strains that exhibit a particular disease trait. The pathophysiology of diseases such as hyperglycemia, hypertension, and hyperlipidemia may affect therapeutic efficacy and toxicity of, for example, candidate drugs intended to lower plasma cholesterol levels or reduce blood pressure. Similarly, a test agent may be more toxic to animals having diabetes or preexisting heart or vascular disease than to normal animals. Thus, parental strains can be selected that have inbred disease conditions such as hypertension, hyperlipemia, diabetes, and renal disease. Suitable strains of rats include, for example, Dahl S rats (hypertension, hyperlipidemia, and insulin resistance), Fawn Hooded hypertension rats (hypertension, pulmonary hypertension, and renal disease), Spontaneously hypertensive rats (hypertension), Stroke prone hypertensive rats (stroke), Zucker obese rats (obesity, hypertension, hyperlipidemia and insulin resistance) and GK rats (type two diabetes and renal disease). Parental strains are selected to achieve the desired level of genetic diversity, while maintaining the chosen disease condition. In other embodiments, one can combinatorially mate parental strains that, collectively, exhibit a plurality of diseases traits.

### Cell lines, tissue banks, and cDNA libraries

The invention also provides cell lines, tissue banks and other materials (e.g., RNA and proteins extracted from various organs and cell types for proteonomics, nuclear magnetic resonance analysis for small molecule identification (metabonomics), and mRNA expression arrays derived from a collection of rats described above. Cell lines can include immortalized cell lines. Tissue banks can include any type of tissue (e.g., kidney, liver, lung, lymphatic, stomach, pancreas, brain, prostate, testis, mammary and ovary). Methods for making cell lines and tissue banks are well known, as are methods for extracting RNA or proteins and for making cDNA and protein libraries.

Expression profiling using nucleic acid microarrays, as described, for example, in U.S. Pat Nos. 6,251,601; 5,800,992 and 5,445,934, can be used to identify genes that are differentially expressed under particular conditions between individuals or F₁ strains in combinatorially bred Panels. Thus, microarrays can be used to identify genes that are differentially expressed in combinatorially bred animals and thereby identify those genotypes that exhibit differences in the potency, efficacy and / or toxicity in response to a particular test agent. The microarrays can consist of collection of cDNA, genomic DNA, or oligonucleotide targets. In some embodiments, protein arrays that include polypeptides, polypeptide fragments or antibodies attached to a solid support can be used. Protein arrays can be used, for example, to examine strain differences in the expression of proteins or phosphorylated signaling proteins. Differences in the profiles of expressed proteins between strains also can be evaluated using mass spectroscopic techniques such as LC/MS, particularly when combined with differentially labeling derivatization techniques to quantitate levels of different classes of proteins.

Each sample to be tested in a microarray can be a sample of tissue from an individual animal. Alternatively, each sample to be tested in a microarray can be pooled from a plurality of animals, e.g., RNA, DNA or protein extracted from equal amounts of liver tissue of six combinatorially bred rats that have been exposed to a test agent.

### Evaluating test agents

Pharmacological and toxicological effects of test agents can be evaluated using a collection of rats described above. Test agents include, for example, candidate therapeutic molecules and putative allergens or toxins in products, food or environmental samples. Evaluating a test agent involves contacting a collection of combinatorially bred animals with a test agent. The method by which a test agent is administered to a combinatorially bred animal depends on the nature of the test agent to be evaluated. Test agents can be administered to a test animal by, for example, topical application, injection, inhalation delivery, oral delivery, nasal delivery, and dietary supplementation. The dose regimen also depends on the nature of the test agent to be evaluated, and can involve a single administration or repetitive administrations of the test agent. Physiological, biochemical, and genetic effects of test agents can be determined by techniques appropriate for the test agent to be evaluated.

Using a collection according to the invention for evaluating tests agents offers advantages over traditional protocols that use outbred or inbred strains. Traditionally, some investigators have favored using outbred strains, such as Sprague Dawley (SD) or Wistar (Wist) rats, to evaluate candidate therapeutic agents and putative allergens or toxins. This is because the individuals that comprise an outbred strain exhibit some degree of genetic heterogeneity, a feature of the human population. However, outbred strains may often be obtained from closed colonies having limited genetic diversity by virtue of allele fixation, which occurs because the number of progenitors establishing a foundation colony is often small and the number of alleles available to transmit to the next generations is limited, See, Festing, M.F., Environ. Health Perspect. 103:44-52, 1995. Thus, outbred strains typically are not as genetically diverse as desired and are not suitable models to represent the scope of human genetic diversity.

The limited genetic heterogeneity provided by outbred rats also comes at the expense of variability in results when evaluating test agents, the inability to replicate experiments between and within laboratories using different groups of rats from the same colony, and difficult authentication and quality control. For example, outbred strains propagated by different suppliers will have different genotypes and often respond differently to various test agents. This phenomenon can leads to unacceptable variability in biochemical and molecular evaluations of test agents. In addition, genetic marker analyses of SD rats indicates an average of only 2 alleles per marker. If this allele frequency is true for each of the 30,000 or so rat genes and a toxic response of a test agent requires the appropriate combination of 2 genotypes, evaluating a test agent in a typical 50-anima! protocol may not reveal this potential effect. The limited genetic diversity indicates that very large sample sizes are needed to accurately evaluate the safety of test agents using outbred strains. Further complicating the use of outbred strains to evaluate test agents is the improbability that an identical collection of animals could be assembled to replicate an observed effect. Finally, the lack of discriminatory genetic markers for outbred strains makes authenticity and quality control for outbred stocks difficult.

Inbred strains typically are created after at least 20 serial brother-sister crosses. The animals that comprise an inbred strain are considered isogenic (i.e., each individual is considered genetically identical). Inbreeding often has been used to create special strains that mimic particular human disease phenotypes. Inbred animals often have been used for evaluating test agents because effects in different individuals within a strain are expected to be similar, with the environment providing the primary variable. The United States National Toxicity Program (NTP) uses Fischer-344 inbred rats and 1360171 hybrid mice to evaluate the safety of test agents.

Using inbred strains can improve reproducibility in evaluating test agents, but at the expense that the selected strain will not have a appropriate genotype to reveal an adverse effect. Unless there is prior knowledge about the ability of a particular strain to reveal effects of a test agent, selecting a single strain limits the potential to detect effects of test agents, and can lead to misleading conclusions. An additional drawback of using inbred strains is that such animals generally are unhealthy, and can exhibit traits that affect drug responses and toxicity. Inbred strains typically produce smaller litters and exhibit many phenotypic abnormalities as a consequence of being genetically homozygous at all loci.

A collection of rats made according to the invention is genetically heterogeneous, and using such a collection rather than one or more inbred strains increases the ability to detect all possible effects of test agents. In addition, F₁ animals derived from the combinatorial mating of inbred parents are generally healthier than inbred animals. Another advantage of using such a collection of animals to evaluate test agents in that linkage disequilibrium mapping can be used to identify genomic loci associated with differences in the effectiveness or toxicity of the test agents between strains. cDNA and protein microarrays can also be used to identify differential expressed genes contributing to differential responses to test agents between strains in the Panel. In addition, the lack of reproducibility associated with the use of outbred stains to evaluate test agents can be avoided because F₁ animals derived from the combinatorial mating can be reproducibly reconstructed by crossing individuals of the appropriate inbred parent strains.

In one preferred version of the present invention, the collection of rodents is made by combinatorially breeding the rat strains described below in the Examples. Specifically, these strains are Wistar Kyoto, Lewis, Fischer 344 and Brown Norway rats. We envision that this specific collection of rodents and collection of cDNA, RNA and proteins derived from these rodents would be useful and suitable in the present invention. By "strain" we mean to include all sub-strains of the preferred strain. For example, by "Brown Norway" we mean to include the sub-strains BN mcwi, BNss, BNhsd.

These strains are all available from many commercial vendors. In a preferred version of the present invention, the Wistar-Kyoto, Lewis, Fischer 344 and Brown Norway parental strains were purchased from Charles River Laboratories (Wilmington, MA).

The invention will be further described in the following examples, which do not limit the scope of the invention described in the claims.

### EXAMPLES

### Example 1 - Selection and combinatorial mating of inbred rats having specific genetic diversity.

This Example describes the selection and combinatorial mating of 4 genetically different inbred parental strains to make a collection of F₁ offspring that exhibit genetic diversity among SSLP markers linked to genes encoding cytochrome P-450 enzymes. The software program ACP Haplotyper was used to facilitate comparative genetic marker analysis of candidate parent strains.

Genetic markers analysis was performed on a 15-strain subset of 48 inbred strains that have been genotyped with microsatellite markers. See, Steen, R.G., et al., Genome Res. 9:AP1-8, 1999, insert. The 15-strain subset was selected to reduce ACP Haplotyper computing time and output complexity. The 14 strains are listed in Table 1.

**Table 1**

| **Abbreviated Name** | **Complete Name** |
|---|---|
| F344 | Fischer F344 |
| BN | Brown Norway |
| WIST | Wistar |
| COP | Copenhagen |
| BDIX | BDIX |
| LE | Long Evans |
| LEW | Lewis |
| WKY | Wistar Kyoto |
| WAG | Wistar Albino Glaxo |
| ACI | ACI |
| DA | Dark-Agouti |
| WF | Wistar Furth |
| PVG | PVG |
| BUF | Buffalo |
| SD | Sprague-Dawley |

The strains in the 15-strain subset were chosen on the basis of the following criteria: 1) commercial availability and maintenance in appropriate isolators for breeding; 2) separation by at least 10 to 15 steps on the rat phylogenetic tree of Canzian, et al., Genome Res. 7:262-267, 1997; 3) frequent usage by the pharmaceutical industry for toxicological studies; 4) historical use in toxicological studies; and 5) susceptibility or resistance to particular classes of test agents. Suitable commercial resources in inbred rats include Charles River Laboratories (Wilmington, MA), Harlan (Indianapolis, IN), Taconic (Germantown, NY), and academic institutions such as the Jackson Laboratory (Bar Harbor, ME). Rat strains often used in the pharmaceutical industry include F344, LEW, WKY, DA, BN, WAG, PVG, BUF and WF. LEW is more susceptible to peroxisomal proliferation than SD, and F344 more susceptible to the nephrotoxic effects of gentamicin than SD.

The four strains selected for combinatorial mating were F344, BN, LEW and WKY. These parental strains were selected so as to maximize diversity at polymorphic markers in genetic proximity to the following cytochrome P-450 enzymes: Cyp1A1, Cyp1A2, Cyp2 family, Cyp3A3, Cyp4A1 and Cyp4A11. SSLP markers in genetic proximity to Cyp loci were identified in Rat Genome Database (Medical College of Wisconsin, Milwaukee, WI), Locus Link (National enter for Biotechnology Information, Bethesda, MD), and in the scientific literature. Using ACP Haplotyper, visual comparisons of markers linked to the coding sequence loci for Cyp1A1, Cyp1A2, Cyp2 family, Cyp3A3, Cyp4A1 and Cyp4A2 were generated. Heterozygous SSLP marker loci are ignored when ACP Haplotyper is used to compare inbred strains. However, the number of heterozygous SSLP marker loci is low in the four selected strains (74085 for BN; 5/4185 for LEW; 3/4224 for F344; and 6/4096 for WKY).

Table 2 shows the number of SSLP markers identified for each group of Cyp loci. By iteratively comparing pairs of strains, the number of different alleles at each Cyp-linked SSLP marker was maximized. Table 2 also shows the number of different alleles in the four selected parent strains for each Cyp locus. In addition, 11 to 19 steps on the rat phylogenetic tree of Conzian, et al. separate each of the selected parent strains from the other three selected strains.

**Table 2**

| | **Cytochrome P450 Loci (CYP)** | | | | | |
|---|---|---|---|---|---|---|
| | 1A1/A2 | 4A1 | 2B2/2A3A | 2C | 4A2 | 3A3 |
| No. SSLP Markers Evaluated | 2 | 3 | 5 | 1 | 2 | 1 |
| Theoretical Maximum No. of Different Alleles in 4 Strains | 8 | 12 | 20 | 4 | 8 | 4 |
| Actual No. of Different Alleles in Selected Strains | 4 | 10 | 9¹ | 2² | 6³ | 3 |
| ¹One locus was not scorable in the BN strain. | | | | | | |
| ²One locus was not scorable in the F344 strain. | | | | | | |
| ³One locus was not scorable in the LEW strain. | | | | | | |

F344, BN, LEW and WKY were obtained from Charles River Laboratories and mated in each possible pair-wise combination (i.e., F344xBN, F344xLEW, F344xWKY, BNxLEW, BNxWKY and LEWxWKY).

Eight offspring from each combinatorial mating of the 4 parental strains were pooled to make a collection of 48 F₁ offspring rats. The collection was housed in a rat containment facility, with each rat housed in a separate cage. Collected F₁ rats are implanted with a microchip for definitive identification, and are genotyped for 1 or more polymorphic markers on each of five different chromosomes. (Example 3, below, describes this particular combination in more detail.)

### Example 2 - Identification of inbred rats having general genetic diversity.

This example describes a comparison of the degree of genetic similarity on a particular chromosome between pairs of rodent strains. Allele sizes for 5,214 SSLP loci were determined for the 15 inbred rat strains described in Example 1. Pairwise comparisons were carried out among the strains, and the percentage of loci that had an identical allele in each pair was calculated for each chromosome. The results for chromosome 1, 5, 8 and 12 are shown in Tables 3-6, respectively, in Fig. 1. Similar comparisons were carried out for the remaining chromosomes. Percent Genetic diversity is calculated from the data by subtracting percent similarity from 100%.

### Example 3 - Selection of the parental strains for breeding

Our goal was to maximize genetic diversity between 4 different inbred parental strains to be used for constructing a preferred combinatorial panel (hereinafter referred to as the Panel). Since the entire genome of the 48 most commonly used strains of laboratory rat had been characterized with ∼4,300 genetic markers, we started with the overall degree of genetic diversity for each inbred strain relative to each other using the software program ACP Haplotyper (created in the Bioinformatics Research Center at the Medical College of Wisconsin). To simply matters, genetic marker analysis was performed on a 15-strain subset of 48 inbred strains based upon the following criteria: 1) commercial availability of the strains; 2) estimated separation of the strains on the phylogenetic tree by at least 10 to 15 steps; 3) current and past use by Pharma for toxicological studies; and 4) diversity at the following cytochrome P-450 enzymes: Cyp1A1, Cyp1A2, Cyp2 family, Cyp3A3, Cyp4A1 and Cyp4A2. Rat strains most often used Pharma include F344, LEW, WKY, DA, BN, WAG, PVG, BUF and WF (M. Festing, personal communication).

The 4 strains selected for combinatorial mating were F344 (Fischer 344), BN (Brown Norway), LEW (Lewis) and WKY (Wistar Kyoto). The BN was selected because it is the strain that has been sequenced by the Rat Genome Sequencing Consortium, it is one of the most genetically distinct rats (Canzian, F., Genome Res. 7(3):262-267, 1997). The F344 strain was chosen because it is the predominant rat model used in protocols by the NTP and several pharmaceutical firms and therefore provides a reference point so that strain comparisons can be made to the existing toxicology database. The remaining two parental strains were selected with the notion to maximize genetic diversity at specific regions (cytochrome P-450s) as well as across the entire genome based on genetic markers and that 11 to 19 steps on the rat phylogenetic tree separate each of the selected parental strains from the other three selected strains.

By counting the number of allelic differences at each of the ∼4300 genetic markers in the genome for each parental rat (BN, LEW, F344 and WKY) and knowing the total number of allelic differences in the rat genome (from the 48 known rat strains), we found that the Panel comprises on average 42% of the known rat diversity, using 6 as the average number of alleles per locus. While we did not reach our initial goal of capturing 50% of the genetic diversity in the rat genome, we may have done as well as possible considering the stringent selection criteria that we established prior to the study, most notably: 1) maximal diversity at the cytochrome P-450s; 2) inclusion of the F344 (used by NTP) and BN (this rat strain is being sequenced) strains; and 3) commercial availability.

Table 7 shows the strain-to-strain comparison at 4 different chromosomes, that collectively represent ∼33% of the genome. These data shows that there is a high level of variation (on average 66-72%) between each parental strain in genetic markers at the chromosomal level. Given that the average polymorphism rate between any two strains is approximately 50% (Steen, R.G., et al., Genome Res. 9(6):AP1-8, 1999, insert), having an average polymorphism rate of 71 % is probably close to the maximum that can be attained with any 4 strains.

**Table 7: Percent chromosomal variation found in Panel rats**

| Hybrid | Chr 1 | Chr 2 | Chr 3 | Chr X | Average |
|---|---|---|---|---|---|
| BN vs. F344 | 76% | 84% | 76% | 60% | 74% |
| BN vs. LEW | 59% | 80% | 77% | 31% | 62% |
| BN vs. WKY | 83% | 86% | 73% | 87% | 82% |
| F344 vs. LEW | 62% | 69% | 48% | 52% | 58% |
| F344 vs. WKY | 76% | 70% | 65% | 79% | 73% |
| LEW vs WKY | 78% | 67% | 65% | 89% | 75% |
| Average | 72% | 76% | 67% | 66% | 71% |

Baseline phenotypic characterization. As part of our studies, we collected baseline data sets for each of the six F₁ strains generated in the Panel, the four parental strains and the CD-IGS outbred strain. For example, we measured growth curves of all six Panel strains from about 4 to 10 weeks of age (Fig. 2). These curves demonstrate considerable diversity in weights among strains, but interestingly the slope is quite similar between the strains. Fig. 2 also demonstrates how comparisons between strains can be used to help the Pharma companies "anchor" the Panel data to their existing data.

Baseline clinical chemistries. The baseline clinical chemistry profiles of the six F₁ strains in the Panel, the outbred CD-IGS, and inbred parental strains used to generate the Panel are summarized in Table 8. They were derived by averaging the measured 8-10 animals in each strain (8-10 weeks of age). Table 8 shows the 20 clinical measures for each strain and we also report the test values for the parental rats and Panel rats as averages of all animals within those groups. If the Panel was similar to the outbred CD-IGS phenotypically, then we would have predicted that the baseline clinical chemistries would not be different.

Using a one way ANOVA, we compared the values obtained for the CD-IGS and the Panel and parental rats (Table 8). As expected, the overall response of different groups of vehicle treated rats was dramatically similar (note that 15 of 20 clinical assays were not different). However, the CD-IGS differed from both the Panel and Parental in measurement of ALP, CA, NA, CL and TRIG. For the other 15 clinical chemistry values, the Panel is identical to values measured in the CD-IGS rats and with published data. Differences in baseline clinical chemistry between the CD-IGS and F₁ strains in the Panel suggests a genetic basis for variation in response.

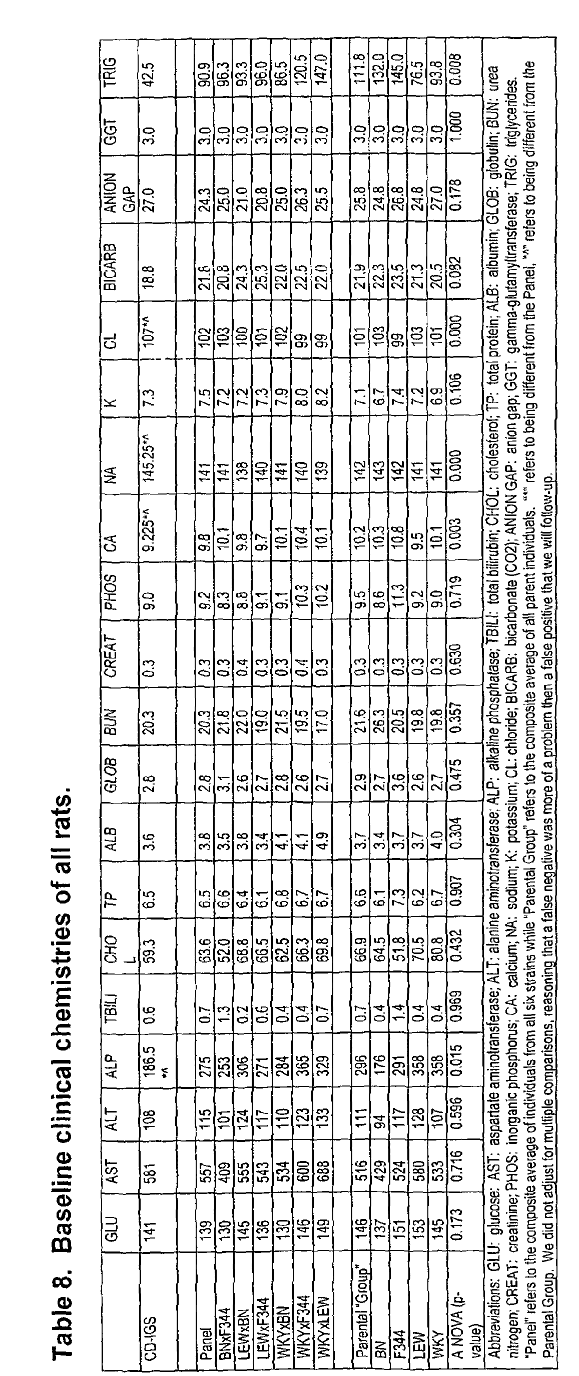

Example 4 - Selection and evaluation of the control strain. Since the CD-IGS (Caesarean-Derived International Genetic Standard) from CRL appears to the dominant outbred strain used in Pharma, we included this strain in all experiments as a second reference point. This strain originated in 1925 by Robert W. Dawley from a hybrid hooded male and a female Wistar rat. This strain was obtained by CRL in 1950 from Sprague-Dawley, Inc. and caesarean re-derived in 1955 from original Charles River Sprague-Dawley (SD) colonies.

We also purchased and used the inbred strains (F344, LEW, WKY, BN) from CRL for the inbred controls. These were the same rat strains used to breed the F₁ hybrid rats for the Panel. The CD-IGS has less genetic diversity than researchers expect and the increased phenotypic variance presents an analytical challenge. Gentamicin was selected as the test agent because it is reported in the literature that Sprague Dawley rats are resistant to the nephrotoxic effects of gentamicin as indicated by appearance of protein in the urine, whereas, the LEW strain is sensitive.

In our studies (Fig. 3), we found that a subgroup (n=7) of CD-IGS rats were highly sensitive to gentamicin, while the majority of the same animals (n=11) were resistant. On average, though, most CD-IGS were resistant to the nephrotoxic effects of gentamicin as compared to the LEW and other rat strains (Fig. 4, below). For example, CD-IGS showed only a 50% increase in plasma BUN levels, an index of renal damage, following exposure to gentamicin. In contrast, LEWxF344, BN, F344, LEW and LEWxBn strains showed 2-3-fold increase in BUN when treated with gentamicin.

How does the investigator best interpret the variability in this result? Is the conclusion that gentamicin has toxic effects in some rats? Or, do we presume outliers are present and then carry out confirmatory studies to convince ourselves otherwise? More importantly, is development of the drug to be halted at this stage because of a toxic response by some individuals? We interpret the data to mean that there will always be a high degree of variability in using CD-IGS rats, but that the extent of variation will differ for every drug trial because of genetic differences in the outbred strain. Unfortunately, the scenario described here seems to be played out too often in human drug trials as well. From a drug discovery perspective this would imply that gentamicin would pass a standard toxicity assay (i.e. those screens looking for adverse effects using CD-IGS or the WKY rats) but later the compound would be found to be nephrotoxic in some subset of human patients carrying genotypes that sensitize individuals to the actions of this drug.

Example 5 - The Panel is effective at detecting adverse effects of nephrotoxins. Gentamicin is the first drug that was tested using the Panel. Gentamicin is still commonly used for the treatment of severe gram-negative bacterial infections (like sepsis or pneumonia) in man when the bacteria is likely resistant to other antibiotics. However, gentamicin is also known to be a nephrotoxin that affects proximal tubules and in some individuals can result in deafness and irreversible vestibular toxicity. We studied gentamicin at 2 doses. The first dose (120 mg/kg) was similar to published dosing regimens (typically 80 mg/kg) that elicit nephrotoxic responses in rats, but this did not produce a robust set of phenotypes, except in LEW rats (not shown). We sought to maximize the range of genetic susceptibility to the adverse drug effects across the strains. Therefore, a higher dose of gentamicin (240 mg/kg) was delivered for 6 consecutive days as an intraperitoneal injection in animals with weights ranging from 225-275g (about 7-10 weeks of age depending on the particular strain).

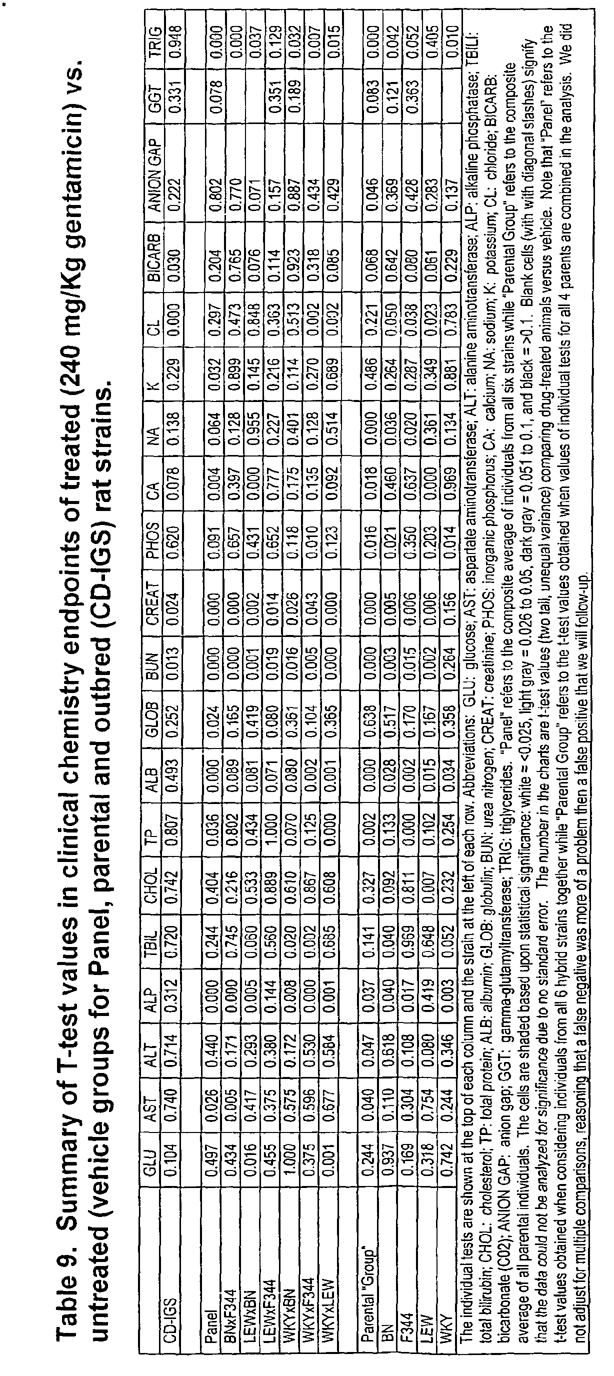

Table 9 summarizes P values for differences in clinical chemistry data between gentamicin and vehicle-treated with strains of rats. The display of the data in this way enables one to quickly identify which clinical chemistry values were influenced by gentamicin in the 6 F₁ Panel strains, 4 parental strains and outbred strain screened in this study. When comparing individuals, all of the strains (except WKY) studied showed a statistically significant increase in either plasma BUN or creatinine concentration (indices of renal damage) when treated with gentamicin (240 mg/Kg/day).

Table 9 also shows that gentamicin (240 mg/Kg/day) also caused significant changes in many other clinical chemistry values. Some of these may not seem relevant at this time, but may be worthwhile exploring in future studies that consider the influence of mixing genetic backgrounds. For example, we found significant changes in 3 indices of liver function, AST (BNxF344), TP (WKYxLEW) and cholesterol (LEW) concentrations in rats treated with the high dose of gentamicin treatment.

Fig. 4 displays the percentage increase in plasma BUN concentration for all of the strains tested in Phase I studies. As described above, on average the CD-IGS appears to be resistant to the nephrotoxic effects of gentamicin relative to the other strain studied. Decoding the combinatorial pattern for the F₁ hybrids suggests that LEW and F344 are the most sensitive strains while WKY is the most resistant. We believe this to be so because in combination with other genotypic backgrounds, WKY is able to either lower (WKYxBN, WKYxLEW) or virtually eliminate (WKYxF344) the susceptibility of BN and LEW genetic backgrounds to the nephrotoxic effects of gentamicin.

A similar pattern is found when analyzing changes in plasma creatinine concentrations between strains. Importantly, after screening the Panel rats and decoding the pattern, we confirmed the sensitivities or resistance of the parental strains in follow-up experiments (also shown in Fig. 4). This means that the data from the combinatorial strategy can accurately predict differences in drug effects in the original parental strains thereby, obviating the need to repeat most drug studies using both the F₁ Panel rats and the parent strains. In addition, this demonstrates that in most cases drug effects will be detected using F₁ hybrids.

Fig. 5 compares the protein excretion for all 11 strains during the first 6 days of treatment with gentamicin. These data are consistent with the BUN and creatinine levels found in the plasma of these same animals and correlates well with histological examination of kidneys for the degree of renal damage in each strain (Fig. 6). For example, the kidneys from LEWxF344 F₁ rats were the most damaged (nearly complete necrosis of all proximal tubules) and they exhibited the largest increase in plasma concentrations of BUN and creatinine. All kidneys from gentamicin-treated parental strains had greater than 50% of tubules damaged, except the WKY. Gentamicin had virtually no effect on the kidney of WKY rats (both vehicle and gentamicin treated animals had kidneys with 25% injured tubules) and gentamicin had the least effect on plasma concentrations of BUN and creatinine and protein excretion in this strain. Histological examination of the kidneys of F₁ hybrids exhibited 50% or more damage to proximal tubules, except in the WKYxBN F₁ strain which exhibited little renal damage and minimal changes in plasma BUN, creatinine concentrations or protein excretion.

The summary of results with gentamicin are consistent with the view that: 1) the Panel is superior to detecting a range of drug effects caused by treatment with gentamicin than either the outbred strain (CD-IGS) commonly used by Pharma or the most commonly used inbred strain (F344) alone; and 2) testing drugs in a combinatorial fashion enables the unmasking of genetic influences on drug responses (pharmacogenomic effects). Quantifying differences in these traits, between strain overlapping them with differences in genetic markers throughout the genome, will facilitate identification of the genes responsible for susceptibility or resistance to the actions of drugs, chemicals and food stuffs, and strain differences in mRNA expression levels using microarrays. 3) The studies with gentamicin demonstrate that a panel of F₁ rat strains is capable of detecting genetic differences in drug responses.

We also performed an additional study with a third compound, cisplatin, that is a commonly used anti-cancer treatment and that also has significant nephrotoxic effects in man (Huang, Q., et al., Toxicol. Sci. 63(2):196-207, 2001; Miura, K., et al., Toxicology 44(2):147-158, 1987; Demeule, M., et al., Am. J. Physiol. 277(6 Pt 2): F832-F840, 1999). At a dose of 5 mg/Kg, all of the cisplatin treated animals responded with significant renal damage (compared to vehicle treated animals) as reflected by elevations in plasma concentrations of BUN and creatinine (Table 10). We found there were marked differences in the degree of proteinuria, increases in glucose excretion and histologic damage to renal proximal tubules between the strains that reflected genetic differences in susceptibility to the nephrotoxic effects of cisplatin.

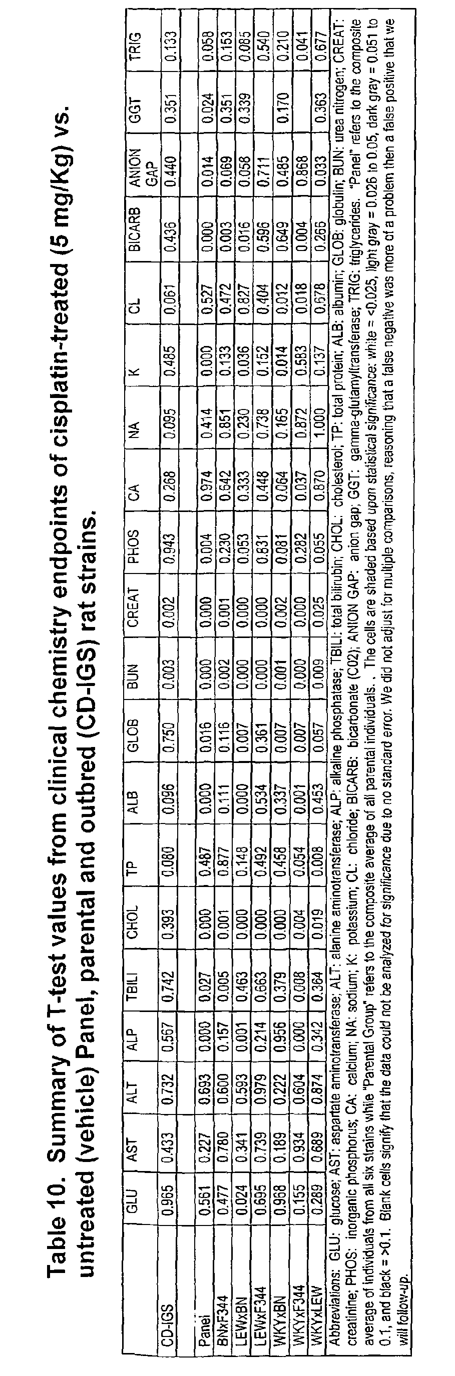

We also detected a significant change in plasma glucose levels in LEWxBN F₁ rats treated with cisplatin that was not seen in any other strain (Table 10). This is of interest since cisplatin is reported to produce hyperglycemia in some patients (Goldstein, R.S., et al., Toxicology 24(3-4):273-80, 1982; Goldstein, R.S., et al., Toxicol. Appl. Pharmacol. 69(3):432-441, 1983; Gogas, H., et al., Gynecol. Oncol. 61 (1):22-26, 1996). Here, only one of the Panel F₁ hybrids (and not the control CD-IGS) detected this similarity to the human clinical response. This data further suggests that the F₁ Panel is superior in detecting genetic differences in drug responses in comparison to using outbred or inbred strains alone.

Example 6 - The Panel can detect differences in the function and tonicity of a hepatotoxin. Clofibrate is a third drug that we have compared clinical chemistry and histopathology responses using the Panel, the parental strains and an outbred strain. Clofibrate is an antilipidemic drug that has been commonly used in Europe, however, it has also been reported to be a hepatotoxin and produce liver cancer in some patients. We delivered the clofibrate orally by gavage (250 mg/Kg in hydroxyproply methyl cellulose) to the rats on 6 consecutive days. Interestingly, Fig. 7 shows that at this dose of clofibrate, the triglyceride levels of most, but not all, strains were significantly lowered. However, the magnitude of the fall in plasma triglyceride levels varied 3-fold between the WKY-derived strains (WKY×LEW, WKY×BN and WKY×F344). BN rats did not exhibit any antilipidemic response to clofibrate and "patients" with BN-like genotypes may be expected to fail to respond therapeutically to this class of compounds. This strongly suggests that researchers can use the Panel to uncover genetic differences in drug responses and the differences in genetic background of the F₁ rats can be exploited to identify the genes involved determining individual response to the therapeutic and toxic effects of various classes of compounds.

The clinical chemistry profiles for the parentals and the Panel did not generate as robust a prediction of liver damage as hoped for at this dose (250 mg/kg clofibrate). For example, AST levels (a predictor of liver toxicity) were not significantly affected in any of the strains (data not shown). However, one strain (the WKY×BN F₁s) did exhibit a significant increase in ALT levels (Fig. 8), demonstrating at least one combination of genotypes in the Panel could detect the known toxicity of this compound. In addition, alkaline phosphatase levels (ALP) another index of liver damage was significant for 5 out of the 6 F₁ hybrid strains, but not altered in the CD-IGS (data not shown).

In contrast, all the strains exhibited hypertrophy of the liver when treated with clofibrate. Hypolipidemic drugs, such as clofibrate, commonly induce peroxisomal proliferation and an increase in the activity of peroxisomal beta-oxidation pathway. We looked for these changes in our rats by histological analysis of a section of the left lateral lobe from livers of either clofibrate or vehicle treated rats. While we could detect cellular differences (e.g. hepatocelluar hypertrophy, hepatocellular vacuolization, anisokaryosis and portal lymphocytic infiltrates) among the clofibrate treated livers versus the vehicle controls within any strain. However, no quantitative differences in the histopathology could be detected between the strains within the Panel.

Since the histological assessment of the liver could not distinguish between the various strains, we turned to measurement of fatty acid oxidase activity to determine genetic differences in the degree of peroxisome proliferation response to clofibrate in the liver between the strains. The assay was done by using a fluorometric assay for rat liver peroxisomal fatty-acyl CoA oxidase activity (FAO). The first step of this assay uses a palmitoyl-CoA dependent NAD+ reducing system to oxidize FAO and produce H₂O₂. The H₂O₂ is then coupled in a peroxidase catalyzed reaction to the oxidation of scopoletin, a naturally fluorescent compound. A fall in fluorescence of the sample indicates an increase in FAO activity which subsequently reflects the increase in the fatty acid oxidase in the liver. We compared the FAO activity found in microsomes prepared from livers of rats treated either with clofibrate or vehicle. We tested one parental strain previously shown to be sensitive to clofibrate (LEW), the resistant outbred (CD-IGS) and three of the F₁ strains in the Panel.

Six clofibrate samples and four vehicle samples were individually tested for FAO activity in duplicate tests. Using an H₂O₂ standard curve enables the change in fluorescence to be converted to FAO activity units (Walusimbi-Kisitu, M. and E.H. Harrison, J. Lipid Res. 24(8):1077-1084, 1983). A significant difference (p=.003) was found by ANOVA analysis of all strains and T-test (two tailed distribution and assuming unequal variance) analysis confirmed that there were significant differences between the strains (not shown). Fig. 9 further illustrates the increase in FAO activities due to clofibrate treatment and shows that three of the Panel strains and the LEW parental are all significantly different from the resistant CD-IGS (significance denoted by an asterisk). This suggests that Panel (and the LEW rat) is a better model for detecting peroxisomal proliferative effects of clofibrate in the liver than the commonly used outbred rat and that CD-IGS rats are resistant to the hepatotoxic effects of clofibrate relative to other strains. Within the Panel, the LEWxF344 hybrid (noted by the cross symbol) was the most sensitive to clofibrate treatment and also shows a significant difference in FAO activity from the two other related hybrid strains (LEW×BN, WKY×LEW). Like the studies with gentamicin, this data reveals that we are better able to detect genetic differences in the response to clofibrate using an F₁ Panel than when using outbred CD-IGS rats or inbred strains alone.

### Example 7 - Preparation of nucleic acid microarrays.

This example describes the preparation of the cDNA microarray used with the Panel to identify differentially expressed genes that contribute to the genetic differences in drug responses among the strains.

A total of 8448 rat UniGenes are obtained from Research Genetics, Huntsville, AL, USA. The UniGene set contains 1928 known genes and 6520 unknown expressed sequence tags. Each nucleic acid in the UniGene set is amplified by PCR with a primer set including T7/T3 promoter: 5'⁻TTACGAATTTAATACGACTCACTATA-3'/5'-AAGCTAAAATTAACCCT CACTAAAGGG-3'. PCR reactions are carried out in 384-well plates with reaction volume of 10 ul using a Pelletier Thermal Cycler PTC-225 (MJ Research, Watertown, AM): 1 ul 10x PCR buffer, 0.5 U Taq DNA polymerase purified by Centri-Sep Column (Princeton Separation, Adelphia, NJ), 250 nM each primer, 200 uM each dNTP, and 0.4 ul liquid culture of each cDNA clone-harboring *Escherichia coli.* PCR cycles are as follows: first cycle, at 95°C for 11 min, at 55°C for 1 minute, and at 72°C for 1.5 minutes; 2^{nd}-36^{th} cycles, at 95°C for 1 minute, at 55°C for 1 minute, and at 72°C for 1.5 minutes; and final extension at 72°C for 7 minutes. After the reaction, 7 ul of water and 17 ul DMSO are added to the reaction mixtures. PCR reactions are electrophoresed on 2% agarose gel and are visualized with an EAGLE EYE II (Strategene).

Microarrays were produced on poly-L-lysine coated slides with a space of 180 um in an area of 18 x 36 mm using a four-pin arraying robot, (Genomic Solution, Ann Arbor, MI). Each gene / EST was stamped from the 384-well plates directly without purification of PCR products. Each gene/EST is stamped in duplicate on the slide, once in a 96 x 96 array on the top and once in a 96 x 96 array on the bottom of the slide. Printed arrays are incubated in a humidity chamber (Sigma, St. Louis, MO) to allow rehydration with lx SSC for 2 hours and heated on hot plates at 85°C for 2 hours. The arrays are UV-crosslinked at 65 mJ with a Stratalinker (Stratagene, La Jolla, CA), stabilized in 10% formalin for 1 min, and rinsed with distilled water twice. The arrays are immersed in 5.5 g succinic anhydride in 325 ml of 1-methyl-2-pyrrolidinone and 25 ml of 1 M sodium borate (pH 8.0) for 20 minutes, then submerged in distilled water for 2 minutes at 95°C, and quickly transferred into 95% EtOH. The arrays are dried by centrifugation and stored in the dark at room temperature.

### Examples 8 - Gene expression profiles.

This example describes the use of DNA microarrays to generate tissue-specific, drug-induced gene expression profiles in combinatorially bred F₁ rats that can be used to identify genes that are differentially expressed and contribute to the differences in drug responses between the strains.

RNA was extracted and processed from the kidney and livers of Panel rats. RNA samples from 8 SD animals exhibiting similar drug responses were pooled. RNA samples from 3 F₁ animals of each strain were pooled mRNA was isolated from total RNA using an Oligotex mRNA Kit (Qiagen, Valencia, CA). Cy3- or Cy5-dUTP (Amersham, Piscataway, NJ) is incorporated into cDNA created from 4 ug mRNA in a 90 ul reaction volume: 9 ul of 10x PCR buffer, 10 mM DTT, 2.5 mM MgC1₂, 4 ug oligo(dT)12-18 primer, 1800 U SuperScriptII (Invitrogen, Carlsbad, CA), 500 uM each dATP, dCTP and dGTP, 40 uM dTTP, 40 uM Cy3- or Cy5-dUTP at 39°C for 2 minutes. 16 U RNAase H (Gibco Invitrogen, Carlsbad, CA) is added, and the reaction mixture is incubated for 30 minutes at 37°C. Cy3- and Cy5-labelled samples are mixed and sequentially purified with a Centri-Sep Column, PCR purification Kit (Qiagen) and concentrated with a Microcon YM-30 (Millipore, Bedford, MA) after the addition of 30 ug of mouse Cotl DNA (Gibco Invitrogen).

To the concentrated probe, 20 ug poly-dA (Sigma) and 40 ug yeast total RNA (Gibco Invitrogen) is added, and 21 ul of the hybridization solution including 3.5x SSC and 0.35% SDS is applied to a nucleic acid microarray. The microarray is prepared according to Example 3 and hybridization is carried out under a 22 x 40 mm cover slip for 1 7 hours at 65°C. The slide is rinsed in 2x SSC/0.1 % SDS for 2 minutes, 0.2x SSC for 2 minutes and then 0. 5x SSC for 2 minutes at room temperature, and finally scanned with a ScanArray 3000 (General Scanning, Watertown, MA) to detect the two-color fluorescence hybridization signals.

Data was collected using Gleams 2.0 software (NuTec Services, Stafford, TX). Each spot was defined by positioning a grid over the array image: the grid is checked manually and adjusted as needed. Signal intensity is determined by subtraction of local background from the mean intensity. Normalization between the dyes was accomplished by normalizing each dye to mean intensities of all genes. A threshold is selected based on a plot of log (Cy3 + Cy5) versus log (Cy3/Cy5). Genes with signal intensity below 20% of the mean of Cy3 intensity plus Cy5 intensity for all genes in each array show a large variance in the ratio of Cy3/Cy5 and are discarded from the analysis. Using this threshold, gene signals are compared among treated F1 offspring are between treated F1 offspring and treated SD rats. For example:

| Microarray Number | Sample |
|---|---|
| 1-3 | AxB F_{1 treated} VS. SD_{treated} |
| 4-6 | AxD F_{1 treated} VS. SD_{treated} |
| 7-9 | AxB F_{1 treated} VS. BxC F₁ treated |
| 10-12 | AxD F_{1 treated} VS. CxD F₁ treated |
| 13-15 | SD_{treated} VS. SD_{control} |

For each set of 3 microarrays, one chip is reverse-labeled with the other fluorescent probe to rule out differences in gene expression due to labeling artifacts. For example, slide 1 has A/B_{treated} cDNA labeled with Cy3 and SD_{treated} labeled with Cy5. Slide 1 is stripped and probed using the same cross-labeled RNA. Slide 2 has SD_{treated} cDNA labeled with Cy3 and A/B_{treated c}DNA labeled with Cy5. This design controls for labeling efficiency and. hybridization efficiency.

One or both drug treatments are evaluated for differential effects (e.g., 2-fold or greater change) on the expression of particular genes represented in the microarray. Observed differences in the gene expression profiles in kidney and liver specimens taken from one treated F1 line relative to other F1 lines and SD rats indicate that within the treated groups, different genotypes within a combinatorially bred collection of F₁ rats can differentiate drug effects.

By comparing the gene expression profiles of RNA extracted from various tissues of drug and vehicle treated combinatorially bred hybrid F₁ rats, the genes responsible for drug effects within strains and differences in therapeutic or toxic effects of drugs between strains can be determined.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A collection of rats comprising combinatorially bred offspring of at least four genetically different inbred rat strains, the inbred strains having been mated in each different pair-wise combination to produce the collection, where the inbred strains comprise the Fischer 344, Brown Norway, Lewis, and Wistar Kyoto strains and said offspring comprise six or more F₁ individuals from each different mating of said inbred strains.

2. The collection of claim 1, wherein the rats comprise combinatorially bred offspring of at least six genetically different inbred rat strains.

3. The collection of claim 2, wherein the collection of rats comprises combinatorially bred offspring of at least eight genetically different inbred rat strains.

4. The collection of claim 3, wherein the rats comprise combinatorially bred offspring of at least ten genetically different inbred rat strains.

5. The collection of any one of the preceding claims, comprising at least seven or more F₁ individuals from each different mating.

6. The collection of any one of the preceding claims, wherein said offspring comprise 12 or more F₁ individuals from each different mating of said inbred strains.

7. The collection of any one of the preceding claims, wherein said collections comprises combinatorially bred offspring of at least 6 genetically different inbred strains, and wherein said offspring comprise 8 or more F1 individuals from each different mating of said inbred strains.

8. The collection of any one of the preceding claims, wherein said inbred strains collectively include at least two alleles for a pre-selected genetic locus.

9. The collection of claim 8, wherein said genetic locus encodes a cytochrome P-450 enzyme.

10. The collection of any one of the preceding claims, wherein said inbred strains are selected to maximize the number of alleles at one or more pre-selected genetic loci.

11. The collection of claim 10, wherein said one or more pre-selected genetic loci encode members of a class of toxicologically relevant enzymes.

12. The collection of claim 11, wherein said enzymes are cytochrome P-450 enzymes.

13. The collection of any one of the preceding claims, wherein at least one of said inbred strains exhibits a particular disease trait.

14. A collection of cDNA molecules made by reverse transcription of RNA isolated from members of a collection of rats, where the collection of rats is as defined in any one of the preceding claims and the cDNA molecules are made from each member of the collection of rats.

15. A cDNA microarray comprising a collection of labeled cDNA molecules made by reverse transcription of RNA isolated from each member of a collection of rats as defined in any one claims 1 to 13, wherein said cDNA molecules are hybridized to gene specific DNA, cDNA, or oligonucleotide targets attached to a single solid substrate in a non-random manner.

16. A method for evaluating a test agent, said method comprising administering said test agent to each member of a collection of rats as defined in any one of claims 1 to 13 and measuring an effect of said test agent on said rats.

17. The method of claim 18, wherein said measuring includes preparing cDNA from RNA isolated from said rats and hybridizing said cDNA to a nucleic acid microarray.

## Patentansprüche

1. Sammlung von Ratten, umfassend kombinatorisch gezüchtete Nachkommen von wenigstens vier genetisch verschiedenen Ratteninzuchtstämmen, wobei die Inzuchtstämme in jeder verschiedenen paarweisen Kombination gekreuzt wurden, um die Sammlung zu erzeugen, wobei die Inzuchtstämme die Stämme Fischer 344, Brown Norway, Lewis und Wistar Kyoto umfassen und die Nachkommenschaft sechs oder mehr F₁-Individuen aus jeder verschiedenen Kreuzung dieser Inzuchtstämme umfasst.

2. Sammlung nach Anspruch 1, wobei die Ratten kombinatorisch gezüchtete Nachkommen von wenigstens sechs genetisch verschiedenen Ratteninzuchtstämmen umfassen.

3. Sammlung nach Anspruch 2, wobei die Sammlung von Ratten kombinatorisch gezüchtete Nachkommen von wenigstens acht genetisch verschiedenen Ratteninzuchtstämmen umfasst.

4. Sammlung nach Anspruch 3, wobei die Ratten kombinatorisch gezüchtete Nachkommen von wenigstens zehn genetisch verschiedenen Ratteninzuchtstämmen umfassen.

5. Sammlung nach einem der vorstehenden Ansprüche, die wenigstens sieben oder mehr F₁-Individuen aus jeder verschiedenen Kreuzung umfasst.

6. Sammlung nach einem der vorstehenden Ansprüche, wobei die Nachkommenschaft 12 oder mehr F₁-Individuen aus jeder verschiedenen Kreuzung der Inzuchtstämme umfasst.

7. Sammlung nach einem der vorstehenden Ansprüche, wobei die Sammlung kombinatorisch gezüchtete Nachkommen von wenigstens 6 genetisch verschiedenen Inzuchtstämmen umfasst, und wobei die Nachkommenschaft 8 oder mehr F₁-Individuen aus jeder verschiedenen Kreuzung der Inzuchtstämme umfasst.

8. Sammlung nach einem der vorstehenden Ansprüche, wobei die Inzuchtstämme als Gesamtheit wenigstens zwei Allele für einen vorab ausgewählten genetischen Locus beinhalten.

9. Sammlung nach Anspruch 8, wobei der genetische Locus ein Cytochrom P-450-Enzym codiert.

10. Sammlung nach einem der vorstehenden Ansprüche, wobei die Inzuchtstämme ausgewählt werden, um die Anzahl an Allelen an einem oder mehreren vorab ausgewählten genetischen Loci zu maximieren.

11. Sammlung nach Anspruch 10, wobei der eine oder die mehreren vorab ausgewählten genetischen Loci Mitglieder einer Klasse toxikologisch relevanter Enzyme codieren.

12. Sammlung nach Anspruch 11, wobei die Enzyme Cytochrom P-450-Enzyme sind.

13. Sammlung nach einem der vorstehenden Ansprüche, wobei wenigstens einer der Inzuchtstämme ein spezielles Krankheitsmerkmal zeigt.

14. Sammlung von cDNA-Molekülen, hergestellt durch reverse Transkription von RNA, die aus Mitgliedern einer Sammlung von Ratten isoliert wurde, wobei die Sammlung von Ratten so ist, wie in einem der vorstehenden Ansprüche definiert, und wobei die cDNA-Moleküle aus jedem Mitglied der Sammlung von Ratten hergestellt werden.

15. cDNA-Mikroarray, umfassend eine Sammlung markierter cDNA-Moleküle, die durch die reverse Transkription von RNA, isoliert aus jedem Mitglied einer Sammlung von Ratten, wie definiert in einem der Ansprüche 1 bis 13, hergestellt wurden, wobei die cDNA-Moleküle an genspezifische DNA-, cDNA- oder Oligonukleotid-Targets, die in einer nicht-zufälligen Weise an ein einzelnes festes Trägermaterial angeheftet sind, hybridisiert werden.

16. Verfahren zur Bewertung eines Testmittels, wobei das Verfahren die Verabreichung des Testmittels an jedes Mitglied einer Sammlung von Ratten, wie definiert in einem der Ansprüche 1 bis 13, und das Messen einer Wirkung des Testmittels auf die Ratten umfasst.

17. Verfahren nach Anspruch 16, wobei das Messen die Herstellung von cDNA aus RNA, die aus den Ratten isoliert wurde, und das Hybridisieren der cDNA an einen Nukleinsäure-Mikroarray beinhaltet.

## Revendications

1. Ensemble de rats comprenant une progéniture combinatoire d'au moins quatre souches consanguines de rat génériquement différentes, les souches ayant été accouplées paire par paire dans chaque combinaison différente pour produire l'ensembles de rats dans lequel les souches consanguines comprennent les souches Fischer 344, Brown Norway, Lewis, et Wistar Kyoto et ladite progéniture comprend au moins six individus ou plus F₁ issus de chaque accouplement différent entre lesdites souches consanguines.

2. Ensemble selon la revendication 1, dans lequel les rats comprennent une progéniture combinatoire d'au moins six souches consanguines génétiquement différentes de rat.

3. Ensemble selon la revendication 2, dans lequel l'ensemble de rats comprend une progéniture combinatoire d'au moins huit souches consanguines génétiquement différentes de rat.

4. Ensemble selon la revendication 3, dans lequel les rats comprennent une progéniture combinatoire d'au moins dix souches consanguines génétiquement différentes de rat.

5. Ensemble selon l'une quelconque des revendications précédentes, comprenant au moins sept individus ou plus F₁ issus de chaque accouplement différent.

6. Ensemble selon l'une quelconque des revendications précédentes, dans lequel ladite progéniture comprend au moins douze individus ou plus F₁ issus de chaque accouplement différent desdites souches consanguines.

7. Ensemble selon l'une quelconque des revendications précédentes, comprenant une progéniture combinatoire d'au moins six souches consanguines génétiquement différentes, et dans lequel ladite progéniture comprend au moins huit individus ou plus F₁ issus de chaque accouplement différent entre lesdites souches consanguines.

8. Ensemble selon l'une quelconque des revendications précédentes, dans lequel lesdites souches consanguines incluent de façon collective au moins deux allèles d'un locus génétique présélectionné.

9. Ensemble selon la revendication 8, dans lequel ledit locus génétique code une enzyme cytochrome P-450.

10. Ensemble selon l'une quelconque des revendications précédentes, dans lequel lesdites souches consanguines sont choisies de façon à augmenter le nombre d'allèles à un ou plusieurs locus génétiques présélectionnés.

11. Ensemble selon la revendication 10, dans lequel ledit au moins un ou plusieurs locus génétique présélectionnés codent des membres d'une classe d'enzymes importants au niveau toxicologique.

12. Ensemble selon la revendication 11, dans lequel lesdits enzymes sont des enzymes cytochromes P-450.

13. Ensemble selon l'une quelconques des revendications précédentes, dans lequel au moins l'une desdites souches consanguines présente un trait pour une maladie particulière.

14. Ensemble de molécules d'ADN complémentaire obtenues par transcription inverse de l'ARN isolé parmi les membres de l'ensemble de rats, dans lequel l'ensemble de rats est tel que défini dans n'importe laquelle des revendications précédentes et les molécules d'ADN complémentaire sont obtenues à partir de chaque membre de l'ensemble de rats.

15. Puce à ADN complémentaire comprenant un ensemble de molécules d'ADN complémentaire marquées obtenues par transcription inverse de l'ARN isolé à partir de chaque membre d'un ensemble de rats tel que défini selon l'une quelconque des revendications 1 à 13, dans laquelle lesdites molécules d'ADN complémentaire sont hybridées à des cibles ADN spécifiques de gène, des cibles d'ADN complémentaire ou des cibles oligonucléotidiques attachées à un unique substrat solide de façon non aléatoire.

16. Procédé pour évaluer un agent-test, comprenant le fait d'administrer ledit agent-test à chaque membre d'un ensemble de rats tel que défini dans l'une quelconque des revendications 1 à 13, et le fait de mesurer un effet dudit agent-test sur lesdits rats.

17. Procédé selon la revendication 16, dans lequel ladite mesure inclut le fait de préparer l'ADN complémentaire à partir d'ARN isolé desdits rats et le fait d'hybrider lesdits ADN complémentaires à une puce d'acide nucléique.
